## Europäisches Patentamt
## European Patent Office
## Office européen des brevets

(19)

(11) Numéro de publication: **0 041 910**
**A2**

(12) # DEMANDE DE BREVET EUROPEEN

(21) Numéro de dépôt: **81420088.7**

(22) Date de dépôt: **04.06.81**

(51) Int. Cl.³: **G 02 C 13/00**
**A 61 B 3/04**

(30) Priorité: **05.06.80 FR 8012809**

(43) Date de publication de la demande:
**16.12.81 Bulletin 81/50**

(84) Etats contractants désignés:
**AT BE CH DE FR GB IT LI**

(71) Demandeur: **Bailly, Jean**
**42, rue Jean Jaurès**
**F-42800 Rive de Gier(FR)**

(72) Inventeur: **Bailly, Jean**
**42, rue Jean Jaurès**
**F-42800 Rive de Gier(FR)**

(74) Mandataire: **Buttet, Roger et al,**
**"Cabinet Charras" 3, Place de l'Hôtel-de-Ville**
**F-42000 Saint-Etienne(FR)**

(54) **Lunette d'essai pour verres correcteurs.**

(57) Lunette d'essai pour verres correcteurs.

L'invention concerne une lunette d'essai pour verres correcteurs, qui permet à l'opticien de faire essayer à son client la correction visuelle envisagée, avant l'équipement définitif, et plus particulièrement dans le cas où sont prévus des verres multifocaux.

Les systèmes connus à ce jour sont insuffisants et incomplets, et répondent aux besoins de seulement 10% des cas.

Selon l'invention, la monture comprend au moins deux drageoirs autorisant le montage des verres ronds calibrés (2) devant chaque oeil, une fente transversale (3) étant réalisée dans l'épaisseur de la monture, au niveau de chaque oeil, pour autoriser à l'encontre des ressorts (4), l'écartement partiel et limité, puis le serrage, des extrémités des branches de la monture, au moins une fente longitudinale (5) étant réalisée dans l'épaisseur de ladite monture, entre les drageoirs, en prenant naissance au niveau de la fente transversale (3) et s'étendant partiellement le long de la monture.

FIG. 2

- 1 -

<u>Lunette d'essai pour verres correcteurs.</u>

La présente invention concerne une monture de lunette en matière plastique, permettant à l'opticien de faire essayer à
son client la correction visuelle envisagée, avant l'équipement définitif, plus particulièrement dans le cas où sont prévus des verres multifocaux.

En effet, les lunettes à double foyers ou à verres progressifs
posent de nombreux problèmes à l'opticien ainsi qu'à l'usager.
Beaucoup de personnes s'adaptent mal à ce genre de verres ou
éprouvent plus ou moins de difficultés pour utiliser correctement leurs lunettes. Il n'est pas rare d'entendre des réflexions
du genre : "Je ne veux pas de double foyers, car j'ai peur de
tomber dans les escaliers".

L'opticien est donc souvent embarrassé pour guider judicieusement son client et l'orienter vers la solution qui serait la
meilleure pour lui, entre les verres simple foyer, double
foyers, triple foyers, ou progressifs. Aussi il est certain que
beaucoup de lunettes sont vendues qui ne représentent pas la
solution optimum pour l'acheteur, ce qui donne lieu à un nombre assez important de réclamations.

Des systèmes ont été imaginés pour permettre au futur porteur
de lunettes, de faire l'essai de ses verres avant leur achat,
mais tous les dispositifs connus sont très incomplets et souvent d'une manipulation malaisée. Les coffrets d'essai existants contiennent tout au plus, une vingtaine de paires de verres
d'essai, alors qu'il en faudrait plusieurs milliers pour couvrir seulement les cas les plus courants. Leur inconvénient
est donc de permettre une démonstration correcte pour un nombre très restreint de personnes : sûrement moins de dix pour
cent des cas. De plus, rien n'existe pour l'essai des triple
foyers.

La lunette d'essai selon l'invention évite ces inconvénients
et permet de faire essayer au future porteur de lunettes, sa
correction optique exacte, soit en simple foyer, en double
foyers, en triple foyers, ou en verres progressifs, et cela
quelle que soit la correction optique nécessaire, dans la
quasi totalité des cas.

Cette lunette est caractérisée en ce que sa monture comprend
un drageoir double (1) qui permet de maintenir devant chaque
oeil, deux verres ronds calibrés (2). L'introduction des verres dans les drageoirs est facilitée par une fente transversale (3) réalisée dans la monture, au niveau de chaque oeil,
les branches extrêmes en regard de ladite monture étant reliées
l'une à l'autre élastiquement par ressort (4) ; un ressort
étant prévu pour chaque drageoir. Il est donc possible d'obtenir un écartement temporaire et limité, et un serrage, entre
les extrémités des branches en regard de la monture. Chaque
verre peut être introduit séparément dans son drageoir, une
fente longitudinale (5) laissant une certaine indépendance de
serrage. Cette fente (5) est réalisée dans l'épaisseur de la
monture, entre les drageoirs, et prend naissance au niveau de
la fente transversale (3) ; elle s'étend partiellement le long
de la monture, entre lesdits drageoirs. Ladite fente (5) combine ses effets avec la fente (3) et permet d'absorber les
différences de dimensions et de calibrage des verres disposés
dans chaque drageoir.

Un marquage angulaire (6) des axes réalisé de 15 degrés en
15 degrés, permet l'orientation judicieuse des verres à correction astigmate.

L'opticien dispose ainsi d'une lunette d'essai qui lui permet permet de placer dans le premier drageoir un verre multifocal et dans le deuxième drageoir un verre complémentaire à simple foyer. La combinaison des puissances disponibles lui permet de couvrir la quasi totalité des diverses corrections optiques possibles, et de pouvoir ainsi faire essayer à son client toutes les solutions envisageables dans son cas particulier. Ce dernier pourra donc se rendre compte par lui-même, des avantages et inconvénients des diverses solutions proposées. Il pourra ainsi choisir en toute connaissance de cause, la formule la mieux adaptée à son cas personnel, en préférant le type de verre qui lui convient le mieux.

La lunette d'essai selon l'invention pourrait comporter trois drageoirs, au lieu de deux, dans le but de couvrir les quelques cas optiques qui peuvent échapper à la lunette à double drageoir, mais à l'usage, il s'avère que cette dernière est suffisante.

Pour permettre une utilisation rationnelle de ces lunettes d'essai, il est prévu trois modèles de coffrets de démonstration, groupant des montures et des verres en nombre variable, suivant les besoins de l'opticien.

I/ le coffret de petite composition qui doit permettre de faire la démonstration souhaitée dans 70% des cas environ, et composé de :
- 6 lunettes d'essai couvrant les écarts pupillaires de 60 à 70 mm, de 2 en 2 mm, soit : 1 lunette d'écart 60 mm + 1 lunette d'écart 62 mm + 1 lunette d'écart 64 mm + 1 lunette d'écart 66 mm + 1 lunette d'écart 68 mm + 1 lunette d'écart 70 mm.
- 4 paires de verres à double foyers couvrant les additions de 1.50 à 3.00 par 0.50, soit :
1 paire de double foyers d'addition 1.50
+ 1 paire de double foyers d'addition 2.00
+ 1 paire de double foyers d'addition 2.50
+ 1 paire de double foyers d'addition 3.00
- 4 paires de verres progressifs couvrant les additions de 1.50 à 3.00 par 0.50, soit :
1 paire de progressifs d'addition 1.50
+ 1 paire de progressifs d'addition 2.00

0041910

+ 1 paire de progressifs d'addition 2.50

+ 1 paire de progressifs d'addition 3.00

- 8 paires de verres sphériques convexes de + 0.50 à + 4.00 par 0.50

- 8 paires de verres sphériques concaves de - 0.50 à - 4.00 par 0.50

- 9 paires de verres cyl (0.50) sphères plan à + 4.00 par 0.50

- 8 paires de verres cyl (0.50) sphères - 0.50 à - 4.00 par 0.50

- 9 paires de verres cyl (1.00) sphères plan à + 4.00 par 0.50

- 8 paires de verres cyl (1.00) sphères - 0.50 à 4.00 par 0.50

II/ le coffret de moyenne composition qui doit permettre de faire la démonstration souhaitée dans 85% des cas environ, et composé de :

- 6 lunettes d'essai couvrant les écarts pupillaires de 60 à 70 mm, de 2 en 2 mm

- 7 paires de verres à double foyers, couvrant les additions de 1.50 à 3.00 par 0.25

- 7 paires de verres progressifs couvrant les additions de 1.50 à 3.00 par 0.25

- 4 paires de verres à triple foyers couvrant les additions de 2.25 à 3.00 par 0.25

- 12 paires de verres sphériques convexes de + 0.25 à + 4.00 par 0.25

- 12 paires de verres sphériques concaves de - 025 à - 4.00 par 0.25

- 13 paires de verres cyl (0.50) sphères plan à + 4.00 par 0.25

- 12 paires de verres cyl (0.50) sphères - 0.25 à - 4.00 par 0.25

- 13 paires de verres cyl (0.75) sphères plan à + 4.00 par 0.25

- 12 paires de verres cyl (0.75) sphères - 0.25 à - 4.00 par 0.25

- 13 paires de verres cyl (1.00) sphères plan à + 4.00 par 0.25

- 12 paires de verres cyl (1.00) sphère - 0.25 à 4.00 par 0.25

III/ le coffret de grande composition qui doit permettre de faire la démonstration souhaitée dans 99% des cas environ, et composé de :

- 13 lunettes d'essai couvrant les écarts pupillaires de 60 à 72 mm, de mm en mm
- 10 paires de verres à double foyers couvrant les additions de 1.25 à 3.50 par 0.25
- 10 paires de verres progressifs couvrant les additions de 1.25 à 3.50 par 0.25
- 7 paires de verres à triple foyers couvrant les additions de 2.00 à 3.50 par 0.25
- 20 paires de verres sphériques convexes de + 0.25 à + 6.00
- 20 paires de verres sphériques concaves de - 0.25 à - 6.00
- 13 paires de verres cyl (0.25) sphères plan à + 4.00
- 12 paires de verres cyl (0.25) sphère - 0.25 à - 4.00
- 21 paires de verres cyl (0.50) sphères plan à + 6.00
- 20 paires de verres cyl (0.50) sphère - 0.25 à - 6.00
- 21 paires de verres cyl (0.75) sphères plan à + 6.00
- 20 paires de verres cyl (0.75) sphères - 0.25 à - 6.00
- 21 paires de verres cyl (1.00) sphères plan à + 6.00
- 20 paires de verres cyl (1.00) sphère - 0.25 à - 6.00
- 13 paires de verres cyl (1.50) sphères plan à + 4.00
- 12 paires de verres cyl (1.50) sphère - 0.25 à - 4.00
- 13 paires de verres cyl (2.00) sphères plan à + 4.00
- 12 paires de verres cyl (2.00) sphère - 0.25 à - 4.00

Toute autre composition des coffrets est naturellement possible à étudier, suivant les désirs particuliers des opticiens.

Il est possible également d'utiliser ces lunettes d'essai pour un autre but que la démonstration des verres multifocaux. Les essais de verres spéciaux pour opérés de la cataracte sont parfaitement possibles, ainsi que les essais de verres prismatiques. De même, cette lunette, équipée de la correction optique convenable peut servir de lunette de prêt ou de dépannage.

0041910

Son emploi est donc très général et peut rendre de multiples services à l'opticien et à ses clients.

0041910

Revendications.

1. Lunette d'essai pour verres correcteurs, caractérisée en ce que la monture comprend un drageoir double (1) permettant le montage devant chaque oeil, de deux verres ronds calibrés (2).

2. Lunette d'essai pour verres correcteurs, caractérisée en ce que la monture comprend un drageoir triple (7) permettant le montage devant chaque oeil, de trois verres ronds calibrés (8).

3. Lunette d'essai selon l'une quelconque des revendications 1 et 2, caractérisée en ce que les drageoirs portent une fente (3) permettant d'augmenter leur dimension, pour faciliter l'introduction des verres (2).

4. Lunette d'essai selon l'une quelconque des revendications 1, 2 et 3, caractérisée en ce que la fente (3) est maintenue par des ressorts (4) et qu'il y a un ressort par drageoir.

5. Lunette d'essai selon l'une quelconque des revendications 1, 2, 3 et 4, caractérisée en ce que la fente longitudinale (5) permet d'introduire chaque verre (2), indépendamment l'un de l'autre.

6. Lunette d'essai selon l'une quelconque des revendications 1, 2, 3, 4 et 5, caractérisée en ce que le marquage angulaire (6) des axes, de 15 en 15 degrés, permet l'orientation des verres à correction astigmate.

7. Jeu de verres d'essai calibrés ronds (2) destinés à être utilisés dans une monture selon l'une quelconque des revendications 1, 2, 3, 4, 5 et 6, caractérisés en ce que leur combinaison entre eux permet de couvrir la quasi totalité des corrections optiques.

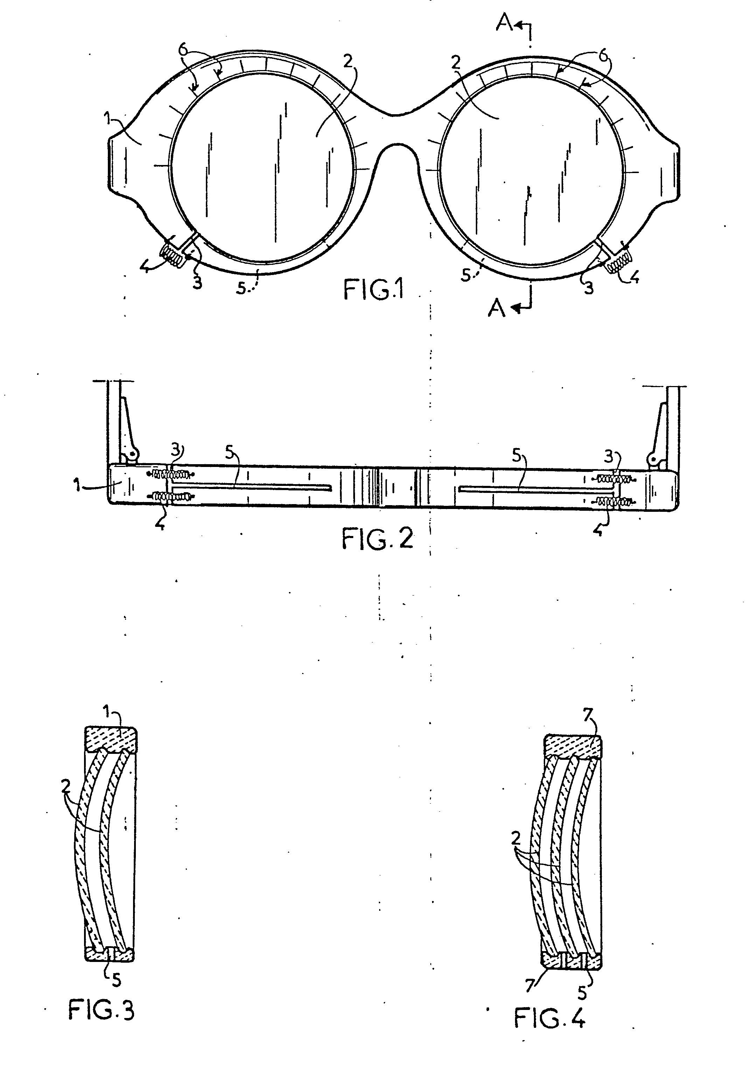

8. Lunette d'essai pour verres correcteurs, caractérisée en ce que la monture comprend au moins deux drageoirs autorisant le montage des verres ronds calibrés (2) devant chaque oeil, une fente transversale (3) étant réalisée dans l'épaisseur de la monture, au niveau de chaque oeil, pour autoriser à l'encontre des ressorts (4), l'écartement partiel et limité, puis le serrage, des extrémités des branches de la monture, au moins une fente longitudinale (5) étant réalisée dans l'épaisseur de ladite monture, entre les drageoirs, en prenant naissance au niveau de la fente transversale (3) et s'étendant partiellement le long de la monture.

0041910

1/1

FIG.1

FIG.2

FIG.3

FIG.4